# EUROPEAN PATENT APPLICATION

(11) **EP 2 644 172 A1**
(43) Date of publication of application: **02.10.2013**
(21) Application number: 13275085.2
(22) Date of filing: 28.03.2013
(51) Int. Cl.: A61F 2/966, A61F 2/962

(54) **Stent holding structure for introducers**

(30) Priority: 30.03.2012 GB 201205719
(71) Applicant: Cook Medical Technologies LLC, Bloomington, IN 47402-0489 (US)
(72) Inventor: Hendriksen, Per, 4160 Herlufmagle (DK); Rasmussen, Erik Edelboe, DK-4200 Slagelse (DK); Dierking, William K., Louisville, KY Kentucky 40222 (US)
(74) Representative: Grainger, David Fraser

(57) **Abstract**

An implantable medical device includes a carrier element having a distal holding member (60) which includes a proximal connecting element (64) which is attached to a guidewire catheter (24) and a distal connecting element (68) which is attached to a dilator tip (20). A proximal connecting element (64) includes a plurality of ribs (74) which have a perpendicular shoulder (80) spaced from an enlarged head (82). The shoulder (80) supports and endmost stent (92) of a device held by the distal holding member (60). Guide wires (98) pass through lumens (86, 88) in the ribs (74) and enlarged head (82), these guidewires (98) being substantially straight. The ribs (74) support an implantable medical device both in the longitudinal direction and in the radial direction. The device (90) can also be pulled rather than pushed by means of the introducer assembly.

## Description

### Technical Field

The present invention relates to a stent holding structure for introducers, to a medical device carrier and to an introducer assembly for the deployment of implantable medical devices such as stents, stent grafts, vena cava filters and so on.

### Background Art

Endoluminally implantable medical devices, such as stents, stent grafts, vena cava filters and the like, tend to be deployed into a patient via an elongate introducer assembly which is passed endoluminally through the vasculature of the patient. For instance, in the case of an aortic implantable medical device, this may be introduced via the femoral artery and passed up to the aorta for deployment. Such introducer assemblies typically comprise at least one catheter or carrier element and an outer protective sheath. They tend to be long, up to 1 to 2 metres overall, flexible yet sufficiently strong to be pushable through tortuous vasculature, as well as resistant to kinking. The implantable medical devices may be expandable by an expansion device such as an inflatable balloon, or self-expandable, being made for instance of an expandable polymer, metal or metal alloy. It is important to hold the implantable medical device securely at the distal end of the introducer assembly to ensure its proper deployment. For this purpose, at the distal end of the introducer assembly there is provided a device carrying assembly which may include a pusher and one or more restraining devices, typically wires. These restraining devices are generally designed to hold the implantable medical device radially compressed against the carrier element even after retraction of the protective outer sheath and until it is determined that the device is precisely positioned and it is appropriate to commence the deployment operation.

The length of the introducer assembly and path it takes through the vasculature of the patient can cause positional difficulties with regard to the device and can cause the device to distort within the assembly. Moreover, where trigger wires are used, these tend to distort as a result of the stresses under which they are put, these stresses being caused by: a) the tendency of the medical device to expand, b) the manner in which the trigger wires are held in the assembly, and c) the operating forces to which they are subjected. Distortion of the trigger wires can lead to movement of the medical device and difficulty in releasing it from the introducer.

Examples of introducer assemblies can be found, for instance, in EP-1,920,739 (also published as US-2009/099637), US-2010/0204770 and WO-2007/100716 (also published as US-7,909,863).

### Disclosure of the Invention

The present invention seeks to provide an improved stent holding structure for introducers, an improved medical device carrier and an improved introducer assembly.

According to an aspect of the present invention, there is provided a medical device carrier for holding an implantable medical device for deployment in a patient, the carrier including: a carrier element on which a medical device can be held, the carrier element including a longitudinal axis and an outer perimeter; at least one rib extending radially outwardly from the perimeter of the carrier element, the at least one rib including a shoulder extending at an angle substantially perpendicular to the longitudinal axis of the carrier catheter, said rib providing a holding shoulder.

The or each rib can be a retention rib.

The or each rib is preferably longitudinal.

The at least one rib (which could also be described as a fin or wing), and in particular the shoulder thereof, provides a stable mechanism for holding the medical device and which, in the preferred embodiment, can allow for some movement of the device during deployment yet without causing the device to lose positional stability or orientation on the carrier. The shoulder, furthermore, can reduce the stresses imparted to any restraining elements, such as trigger wires, leading to easier and more reliable release of the device from the carrier during the deployment operation. Easier release can limit or reduce the chance of jolting of the introducer assembly during deployment, as can happen when it is necessary to exert a large force, particularly to start the deployment procedure.

Advantageously, the medical device carrier includes a plurality of ribs extending radially outwardly from the carrier element. The ribs are preferably angularly spaced around the perimeter of the carrier element, this spacing providing recesses for receiving at least one portion of a medical device to be held on the carrier. A plurality of such ribs can provide multiple support points for the medical device, thus further securing the device to the carrier.

The ribs are preferably substantially evenly spaced angularly around the perimeter of the carrier element.

In the preferred embodiment, the medical device carrier or carrier element includes a distal end for insertion into a patient and a proximal end, wherein the or at least one said shoulder faces the distal end of the medical device carrier or carrier element. Having the shoulder or shoulders face forwardly (distally), they act to pull the medical device rather than compress it, as occurs with a traditional pusher element. Pulling the device can reduce the chance of distortion of the device by squashing.

Advantageously, the medical device carrier includes a bore, channel or groove in the rib or ribs; the or each bore, channel or groove preferably extending in a direction substantially parallel to the longitudinal axis of the carrier element. Providing the bores, channels or grooves in this orientation can ensure that any trigger wires positioned in the bores or channels will remain substantially straight, rather than bending, making it easier to remove the trigger wires at the time of deployment of the device. Easier release (deployment) reduces the chance of jolting the introducer assembly during the procedure and therefore the chance of displacing the medical device.

Preferably, the or each bore, channel or groove is located in the rib or ribs at a position substantially furthest from the carrier element.

Preferably, the rib or ribs include a tapering or chamfered proximal end surface.

In an embodiment, the medical device carrier includes an enlarged diameter head adjacent to the carrier element, the enlarged diameter head facing the shoulder or shoulders of the rib or ribs. This arrangement of the enlarged head provides a space for receiving a part of the medical device within the radial profile of the device carrier.

The enlarged diameter head may include one or more bores or recesses facing the rib or ribs, the or each bore or recess of the enlarged head preferably facing a corresponding bore, channel or groove of a respective rib. The bore or recess can hold the extremity of a trigger wire, thus to provide a holding element to hold a part of the medical device in the space between the enlarged head and shoulders.

Preferably, the or each holding shoulder provides for pulling a medical device endoluminally through a patient during deployment.

According to another aspect of the present invention, there is provided an introducer assembly including: a medical device carrier for holding an implantable medical device for deployment in a patient, the carrier including: a carrier element provided with a carrier section on which a medical device can be held, the carrier element including a longitudinal axis and an outer perimeter; at least one rib extending radially outwardly from the perimeter of the carrier element, the at least one rib including a shoulder extending at an angle substantially perpendicular to the longitudinal axis of the carrier catheter, said rib providing a holding shoulder; and at least one trigger wire.

According to an aspect of the invention, there is provided an introducer assembly including: a medical device carrier for holding an implantable medical device for deployment in a patient, the carrier including: a carrier element on which a medical device can be held, the carrier element including a longitudinal axis and an outer perimeter; at least one rib extending radially outwardly from the perimeter of the carrier element, the at least one rib including a shoulder extending at an angle substantially perpendicular to the longitudinal axis of the carrier catheter, said rib providing a holding shoulder; and at least one trigger wire. Advantageously, the trigger wire or wires pass through channels, bores or grooves in the rib or ribs.

Preferably, the rib or ribs is/are longitudinal.

In one embodiment, the introducer assembly includes an enlarged diameter head facing the rib or ribs, the enlarged diameter head including one or more bores or recesses providing a tight fit of a trigger wire.

In the preferred embodiment, the trigger wire or wires are substantially straight through an associated bore, channel or groove in the rib or ribs and preferably also the respective bore or recess in an enlarged diameter head.

In one embodiment, the introducer assembly includes a plurality of said ribs angularly spaced around the perimeter of the carrier element, said spacing providing recesses for receiving at least one portion of a medical device to be held on the carrier.

There is advantageously provided a sheath arranged to overlie the medical device carrier and movable longitudinally relative thereto. The rib or ribs are preferably a snug fit in the sheath.

In the preferred embodiment, the or each holding shoulder provides for pulling a medical device endoluminally through a patient during deployment, the introducer assembly being provided with no pusher element.

In a preferred embodiment, the or each holding shoulder provides for pulling a medical device during deployment, the introducer assembly being provided with no pusher element.

### Brief Description of the Drawings

Embodiments of the present invention are described below, by way of example only, with reference to the accompanying drawings, in which:
Figures 1 and 2 show an embodiment of a known deployment device;
Figure 3 is an enlarged view of the distal end of a preferred embodiment of carrier element and holder device;
Figure 4 is a longitudinal cross-sectional view of the carrier element and holder device of Figure 3;
Figure 5 is a view of the carrier element and holder device of Figure 3, showing a part of a stent attached thereto;
Figure 6 is a view of the distal end of the introducer assembly according to the preferred embodiment, showing the distal end of the stent carrier element partially exposed beyond the sheath; and
Figure 7 shows the distal end of the carrier element of Figure 6 completely exposed and the stent held thereby in a partially deployed state.

### Description of the Preferred Embodiments

It is to be appreciated that the drawings of this application are intended to be schematic and do not show the components of the introducer assembly to their intended scale. In Figures the components are shown in enlarged form for the purposes of clarity only.

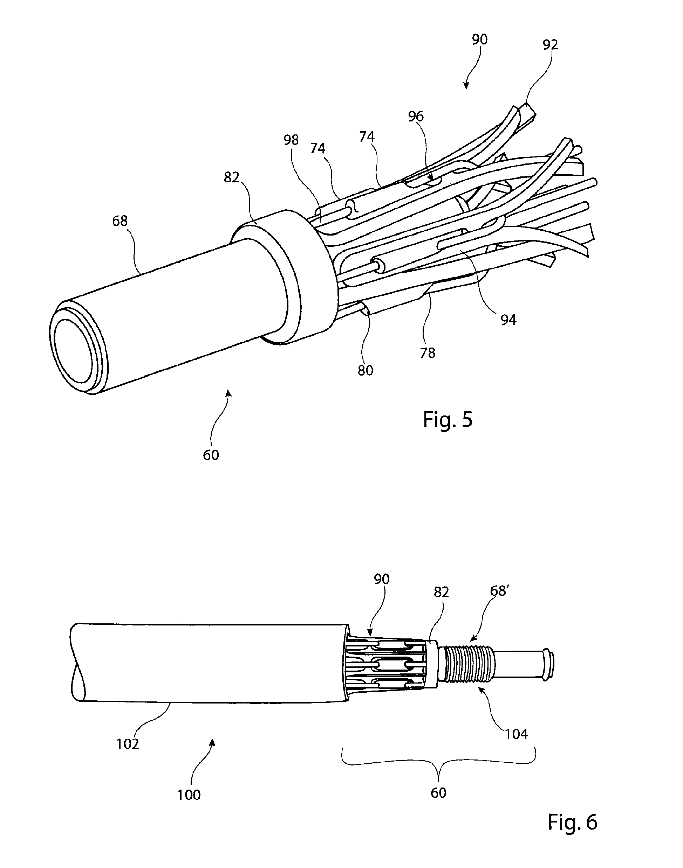

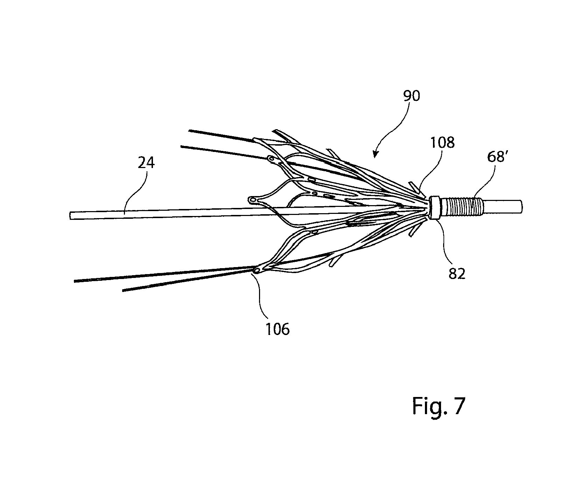

Figures 6 and 7 are based on photographs, the full versions of which can be found in British patent application no. GB1205719.6, from which this application claims priority and which is incorporated herein by reference.

It is also to be appreciated that although the embodiments described below focus on the deployment of a stent, the structure of introducer assembly taught herein can be used for the deployment of a wide variety of medical devices including but not limited to stents, stent grafts, vena cava filters, occluders and so on.

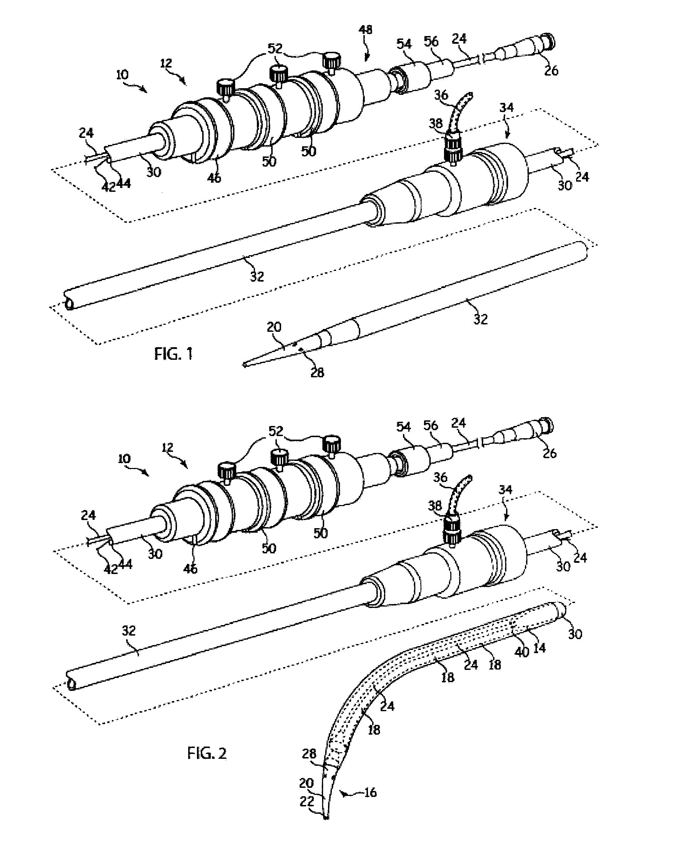

Referring to Figures 1 and 2, the example of known introducer assembly 10 shown includes an external manipulation section 12, a distal attachment region 14 and a proximal attachment region 16. The distal attachment region 14 and the proximal attachment region 16 secure the distal and proximal ends of an implantable medical device 18, respectively. During the medical procedure to deploy the implant 18, the distal and proximal attachment regions 14 and 16 will travel through the patient's lumen to a desired deployment site. The external manipulation section 12, which is actuated by a surgeon to manipulate the introducer, remains outside of the patient throughout the procedure.

The proximal attachment region 16 of the introducer assembly 10 includes a dilator tip 20, which is typically provided with a bore 22 therein for receiving a guide wire (not shown) of conventional type. The longitudinal bore 22 also provides a channel for the introduction of medical reagents. For example, it may be desirable to supply a contrast agent to allow angiography to be performed during the placement and deployment phases of the medical procedure.

A guide wire catheter 24, conventionally made from a flexible thin walled metal tube, is fastened to the dilator tip 20. The guide wire catheter 24 is flexible so that the introducer 10 can be advanced along a relatively tortuous vessel, such as a femoral artery, and so that the distal attachment region 14 can be longitudinally and rotationally manipulated. The guide wire catheter 24 extends through the introducer 10 to the manipulation section 12, terminating at a connection device 26, in conventional manner.

The connection device 26 is designed to accept a syringe to facilitate the introduction of reagents into the inner catheter 24. The guide wire catheter 24 is in fluid communication with apertures 28 in the flexible dilator tip 20. Therefore, reagents introduced into connection device 26 will flow to and emanate from the apertures 28.

A pusher sheath or rod 30 (hereinafter referred to as a pusher member), typically made from a plastics material, is mounted coaxially with and radially outside of the guide wire catheter 24. The pusher member 30 is "thick walled", that is the thickness of its wall is preferably several times greater than that of the guide wire catheter 24.

A sheath 32 extends coaxially over and radially outside of the pusher member 30. The pusher member 30 and the sheath 32 extend distally to the manipulation region 12.

The implantable medical device 18, which may be a stent, a stent graft or any other implant or prosthesis deliverable by this device 10, is retained in a compressed condition in the sheath 32. The sheath 32 extends distally to a sheath manipulator and haemostatic sealing unit 34 of the external manipulation section 12. The haemostatic sealing unit 34 includes a haemostatic seal (not shown) and a side tube 36 held to the unit 34 by a conventional luer lock 38.

The sheath manipulator and haemostatic sealing unit 34 also includes a clamping collar (not shown) that clamps the sheath 32 to the haemostatic seal and a silicone seal ring (not shown) that forms a haemostatic seal around the pusher rod 30. The side tube 38 facilitates the introduction of medical fluids between the pusher rod 30 and the sheath 32. Saline solution is typically used.

During assembly of the introducer assembly 10, the sheath 32 is advanced over the proximal end of the dilator tip 20 of the proximal attachment region 16 while the implant 18 is held in a compressed state by an external force. A suitable distal attachment (retention) section (not visible in Figures 1 and 2) is coupled to the pusher rod 30 and retains a distal end 40 of the prosthesis 18 during the procedure. The distal end of the prosthesis 18 is provided with a loop (not shown) through which a distal trigger wire 42 extends. The distal wire also extends through an aperture (not shown in Figures 1 and 2) in the distal attachment section 40 into an annular region 44 between the inner catheter 24 and the pusher rod 30. The distal trigger wire 42 extends through the annular space 44 to the manipulation region 12 and exits the annular space 44 at a distal wire release mechanism 46.

A proximal portion of the external manipulation section 12 includes at least one release wire actuation section 50 mounted on a body 48, in turn mounted onto the pusher member 30. The guide wire catheter 24 passes through the body 48. The distal wire release mechanism 46 and the proximal wire release mechanism 50 are mounted for slidable movement on the body 48.

The positioning of the proximal and distal wire release mechanisms 46 and 50 is such that the proximal wire release mechanism 46 must be moved before the distal wire release mechanism or mechanisms 50 can be moved. Therefore, the distal end of the implant 18 cannot be released until a self-expanding zigzag stent thereof has been released. Clamping screws 52 prevent inadvertent early release of the prosthesis 18. A haemostatic seal (not shown) is included so that the release wires can extend out through the body 48 without unnecessary blood loss during the medical procedure.

A proximal portion of the external manipulation section 12 includes a pin vise 54 mounted onto the proximal end of the body 48. The pin vise 54 has a screw cap 56. When screwed in, vise jaws (not shown) of the pin vise 54 clamp against or engage the guide wire catheter 24. When the vise jaws are engaged, the guide wire catheter 24 can only move with the body 48 and hence it can only move with the pusher member 30. With the screw cap 56 tightened, the entire assembly can be moved together as one piece.

Once the introducer assembly 12 is in the desired deployment position, the sheath 32 is withdrawn to just proximal of the distal attachment section 14. This action releases the middle portion of the implant 18, in this example a stent or stent graft, so that it can expand radially. Consequently, the stent or stent graft 18 can still be rotated or lengthened or shortened for positioning purposes. The proximal end self-expanding stent however, is still retained at the dilator tip 16 by means of the release wires. Also, the distal end of the stent or stent graft 18 will still retained within the sheath 32.

Next, the pin vise 54 is released to allow small movements of the guide wire catheter 24 with respect to the pusher rod 30 to allow the stent or stent graft 18 to be lengthened, shortened, rotated or compressed for placement in the desired location within the lumen. X-ray opaque markers (not shown) may be placed along the stent or stent graft 18 to assist in the positioning of the prosthesis.

When the proximal end of the stent or stent graft 18 is in place, the proximal trigger wire is withdrawn by distal movement of the proximal wire release mechanism. The proximal wire release mechanism 50 and the proximal trigger wire can be completely removed by passing the proximal wire release mechanism 50 over the pin vise 54, the screw cap 56 and the connection unit 26.

Next, the screw cap 56 of the pin vise 54 is loosened, after which the inner catheter 24 can be pushed in a proximal direction to move the dilator tip 20 in a proximal direction. When the dilator tip 20 no longer surrounds the end of the stent or stent graft 18, it expands to engage the lumen walls of the patient. From this stage on, the proximal end of the stent or stent graft 18 cannot be moved again.

Once the proximal end of the stent or stent graft 18 is anchored, the sheath 32 is withdrawn distally of the distal attachment section 14, which withdrawal allows the distal end of the stent or stent graft 18 to expand. At this point, the distal end of the stent or stent graft 18 may still be repositioned as needed.

Problems can arise with the deployment of an implantable medical device by a mechanism which pushes the device during the procedure. These can include distortion of the device by twisting, compression and so on, leading to potential incorrect positioning and deployment. Problems can also arise if a large force must be used to effect the deployment operation.

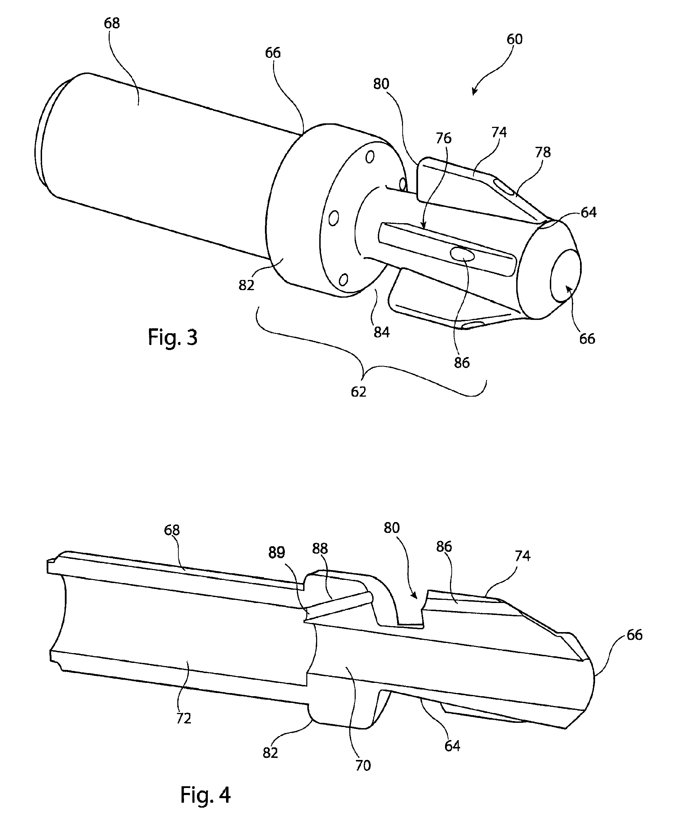

Figures 3 to 7 show a preferred embodiment of medical device carrier or carrier element for deploying an implantable medical device.

Referring first to Figure 3, this shows a portion of the distal end of the medical device carrier and in particular a carrier element or distal holding member 60 thereof, according to the preferred embodiment. The distal holding member 60, which is preferably formed as a single molded unit, is located between guide wire catheter 24 and the dilator tip 20. More specifically, the distal holding member 60 includes a proximal portion 62 which includes a proximal cylindrical connecting element 64 provided with a bore 70 therein opening at the proximal end 66 of the member 60. The distal end of the connecting member 60 includes a second cylindrical connecting element 68 which is received in the dilator tip 20. The connecting element 68 also has a bore 72 therein. The bores 70, 72 of the connecting elements 64 and 68, which can be seen in Figure 4, provide a continuous lumen through the holding member 60, thus allowing passage of a guide wire therethrough. In the embodiments shown in Figures 3 to 7, the connecting element 68 has a larger diameter than the connecting element 64 but this is not necessary or relevant to the disclosures herein. They could have the same or other different relative diameters.

Disposed radially around the proximal connecting element 64 are a plurality of longitudinal ribs or fins 74, substantially evenly angularly spaced around the connecting element 64. These ribs or fins 74 are preferably formed integrally with the connecting element 64 but could equally be bonded or otherwise attached thereto.

The fins or ribs 74 have a narrow width 76 which, as will become apparent from the description below, are able to accommodate an apex portion of an end-most stent to be carried by the introducer assembly. The ribs 74 also have a chamfered or angled trailing (proximal) edge 78 which may, for example, be at an angle of 20-30° to the surface of the tubular connecting element 64 and, in practice, of the longitudinal axis of the holding member 60. The trailing edge 78 could be at other angles, up to and in some instances exceeding 50°.

The distal or leading end 80 of the rib 74 is at an angle which is substantially perpendicular to the longitudinal axis of the holding member 60 and in practice of the surface of the cylindrical connecting element 64, thereby to provide a stop shoulder for purposes described below.

The rib or ribs 74 terminate short of the end of the connecting element 64 with which they are integral and in particular short of an enlarged diameter head 82 of the holding member 60, so as to provide a gap 84 between the shoulders 80 and the enlarged head 82.

The embodiment of Figure 3 has six ribs 74 substantially equally angularly spaced around the cylindrical connecting element 64, although the number of ribs 74 will be dependent upon the design of the device to be restrained by the holding member 60 and the manner in which the medical device is so held. This will become more clear below in connection with the description relating to Figures 6 and 7.

As can be seen in particular with regard to Figures 3 and 4 when viewed together, there is a channel or lumen 86 extending through each rib 74 at a radial location which is substantially furthest from the surface of the cylindrical connecting element 64, proximate what could be described as the radial extremity of each rib 74. In the embodiment shown, each channel or lumen 86 is at a slight angle, extending or tapering towards the cylindrical connecting element 64 in the distal direction of the holding member 60, as will be apparent in particular from the cross-sectional view of Figure 4. In other embodiments, the lumens 86 could be substantially parallel to the surface of the connecting element 64 and in particular to the longitudinal axis thereof. The enlarged head 82 also includes a plurality of channels or bores 88 which are substantially radially aligned with respective lumens 86 in the ribs 74 as will be apparent, for example, from Figures 3 and 4. The lumens 88 in the enlarged head 82 are, in the embodiment shown, oriented at a greater angle to the longitudinal axis of the distal holding member 60, in this example at an angle of around 25°. In practice, this angle could be anything from 0°, that is parallel to the longitudinal axis of the holding member 60, up to an angle of around 30° or even 40° thereto.

The bores 88 are shown extending throughout the entire length of the enlarged head portion 82 and into the lumen 72 of the distal connecting element 68. However, in other embodiments, the lumens 88 could be blind bores, that is being closed at their distal ends 89.

The channels 86 preferably have an inner diameter which is larger than the diameter of the trigger wires used in the assembly, while the bores 88 preferably have an inner diameter which is smaller than the diameter of the distal ends of the trigger wires such that the latter are a tight fit in the bores 88.

The proximal connecting element 64 has an outer diameter which is in practice not significantly greater than the outer diameter of the guide wire catheter 24 and in some embodiments may have the same diameter, being formed integrally with the carrier catheter 24.

Referring now to Figure 5, the distal holding member 60 is shown with the distal end of a stent 92 held therewithin. For the sake of clarity, the guidewire catheter 24, which acts in this embodiment also as the device carrier, is not shown in Figure 5, nor is the dilator tip 20, which would be fitted over the distal connecting element 68.

The medical device 90 includes an end-most stent 92 having a plurality of apex sections 94 which may be formed, for example, from a pair adjacent struts which are connected to one another at an apex, or as an apex portion provided with a slot therein sized to receive a respective rib 74. In this particular example, the end-most stent 92 is of the latter design, whose slots 96 can be seen in Figure 5. Passing through the lumens 86 and bores 88 are trigger wires 98 which also feed into the carrier catheter 24 via suitable apertures or holes (not shown) towards the proximal end of the introducer assembly.

As can be seen in Figure 5, the trigger wires 98, which may be of conventional form, are substantially straight across the ends 94 of the stent 90 as well as being substantially straight between the ribs 74 and enlarged head 82. This differs from existing device holding arrangements using trigger wires, in which the wires are typically bent or relatively sharply curved at the distal end of the device they hold, as the result of their need to constrain the device radially against the carrier catheter and also as a result of the radial expansion force produced by the stent of the medical device in seeking to regain its non-contracted radius. Moreover, such trigger wires, in conventional structures of introducer assemblies, are intended to restrain the device both against radial expansion as well as against longitudinal movement along and rotation around the carrier catheter 24. As a result, the trigger wires in conventional assemblies have to counter forces in a plurality of directions at the point at which the stent or other medical device is held.

By contrast, with the structure of distal holding member 60 taught herein, the shoulders 80 of the ribs 74 provide the support and stop elements for resisting forces in the longitudinal direction of the distal holding member, and thus of the introducer assembly, in a direction towards the proximal end thereof. These forces are particularly prevalent when the introducer assembly is passed through tortuous vasculature and when the outer cover sheath is removed to expose the medical device prior to deployment. In addition, the side walls of the ribs 74, which are preferably substantially parallel to one another and extend substantially radially outwardly from the centre point of the cylindrical connecting element 64, provide angular force support to a stent held by the ribs 74. Thus, this structure and shape of the ribs 74 provides the strength and mechanism to hold a stent of a medical device both in longitudinal and angular position on the holding member 60. As the shape of the shoulders 80, being substantially perpendicular to the longitudinal axis of the distal holding member 60, counters the forces which are imparted particularly during withdrawal of the outer sheath, there is less requirement for the other components of the introducer assembly to resist that force, the same applying to the angular forces which the ribs 74 overcome. As a result, the trigger wires 98 are only required to resist the radial opening force of the stent or other device held thereby. This radial opening force of the stent is generally much less than the other forces to which the stent or medical device is subjected during the deployment procedure, particularly in the case of devices made of shape memory materials designed with a transition point at around body temperature.

These features provide a number of advantages over prior art structures, including the following. The ribs provide a solid support for a stent or other implantable medical device on the carrier element, ensuring proper retention of the device with no undesired migration or distortion of the device during the deployment process. Furthermore, there is less stress imparted to the trigger wires, which makes it easier for them to be removed, that is pulled back, in order to deploy the stent or other device. As a result, as is shown in the preferred embodiment, the trigger wires 98 can also be kept substantially straight, making it easier for them to be withdrawn, thus reducing the force which has to be applied by a clinician in order to release the medical device from the carrier catheter. In this regard, it will be appreciated that in the preferred embodiment, the introducer assembly has no pusher element and relies solely on the or each holding shoulder pulling the medical device during deployment.

Referring now to Figure 6, there is shown a side elevational view of an example of introducer assembly 100, having a distal holding member 60 of the type taught herein. The introducer assembly 100 includes an outer sheath 102, of conventional form, within which the device carrier is held. In this example, distal connecting element 68' has a threaded portion 104 which threads into a dilator 20, the latter being provided with a complimentary internal threaded bore.

As can be seen, the implantable medical device 90 is held radially constrained within the sheath 104. In this example, the sheath 102 is responsible for holding the distal end of the implantable medical device 90, that is the end which is opposite the end held by the holding member 60.

The rib or ribs 74 are a snug fit in the sheath 104.

When the sheath 102 is withdrawn, the distal end 106 of the stent 90 is able to expand radially outwardly, as shown in Figure 7. The proximal end of the stent 90, however, remains attached to and restrained by the distal holding member 60 by virtue of the trigger wires 98. In this example, as can be seen in particular in Figure 7, the stent 90 is provided with a plurality of distally positioned barbs 108, which are not in this example held down by trigger wires 98 but which are able to expand radially outwardly to a certain extent, in this example being limited only by being coupled to the distal structure of the stent 90. The barbs 108 are, nevertheless, able to expand somewhat and will in practice be the first elements of the proximal end of the stent 90 to come into contact with the vessel wall, so as to anchor the stent 90 properly to the vessel before the stent fully deploys.

In the configuration shown in Figure 7, it is possible to correct the position of the stent 90 prior to releasing it fully from the device carrier. For example, the stent may be moved in a longitudinal direction for precise positioning. This is possible because the distal barbs are not yet in engagement with the vessel wall.

Once the distal end 106 of the stent 90 has been fully deployed and once it has been determined that the stent 90 is in the correct position within the patient's vessel, the trigger wires 98 can be withdrawn so as to release the distal struts 94 from the distal holding member 60.

This embodiment, in which the distal end 106 of the medical device 90 is not held by any element of the introducer assembly, avoids any longitudinal compression of the stent 90 during the deployment procedure. Whilst this is considered an important advantage in many cases, it is not excluded that some embodiments may also provide a mechanism for radially constraining (holding) the distal end 106 of the stent 90 until it is desired to expand the distal end 106. Any suitable mechanism, such as trigger wires, retaining caps or collars and so on may be used. It is also envisaged that there could be provided a set of ribs at the distal end of the device to restrain that end in similar manner to its proximal end. These mechanisms will be apparent to the skilled person from knowledge in the art and from the teachings herein.

It will be appreciated that the number and size of the ribs 74 will be dependent upon the design of the medical device to be fitted on the device holding member 60 and that these features are not critical to the performance of the teachings herein.

Although the embodiments described above provide a lumen or channel 86 in the ribs 74, it is to be appreciated that there may be provided an open longitudinally extending groove instead.

All optional and preferred features and modifications of the described embodiments and dependent claims are usable in all aspects of the invention taught herein. Furthermore, the individual features of the dependent claims, as well as all optional and preferred features and modifications of the described embodiments are combinable and interchangeable with one another.

The disclosures in British patent application number GB1205719.6, from which this application claims priority, and in the abstract accompanying this application are incorporated herein by reference.

## Claims

1. A medical device carrier for holding an implantable medical device for deployment in a patient, the carrier including:
a carrier element on which a medical device can be held, the carrier element including a longitudinal axis and an outer perimeter;
at least one longitudinal rib including a bore, channel, or groove therein for receiving a trigger wire, the at least one rib extending radially outwardly from the perimeter of the carrier element and including a distal end and a holding shoulder at the distal end extending at an angle substantially perpendicular to the longitudinal axis of the carrier catheter.

2. A medical device carrier according to claim 1, including a plurality of ribs extending radially outwardly from the carrier element; wherein said ribs are preferably angularly spaced around the perimeter of the carrier element, said spacing preferably providing recesses for receiving at least one portion of a medical device to be held on the carrier.

3. A medical device carrier according to claim 2, wherein the ribs are substantially evenly spaced angularly around the perimeter of the carrier element.

4. A medical device carrier according to any preceding claim, wherein the medical device carrier includes a distal end for insertion into a patient and a proximal end, wherein the or at least one said shoulder faces the distal end of the medical device carrier.

5. A medical device carrier in according to any preceding claim, wherein the or each bore, channel or groove extends in a direction substantially parallel to the longitudinal axis of the carrier element; and/or wherein the or each bore, channel or groove is located in the rib or ribs at a position substantially furthest from the carrier element.

6. A medical device carrier according to any preceding claim, wherein the rib or ribs include a tapering or chamfered proximal end surface.

7. A medical device carrier according to any preceding claim, including an enlarged diameter head adjacent to the carrier element, said enlarged diameter head facing the shoulder or shoulders of the rib or ribs.

8. A medical device carrier according to claim 7, wherein the enlarged diameter head includes one or more bores or recesses facing the rib or ribs; and the or each bore or recess of the enlarged head preferably faces a corresponding bore, channel or groove of a respective rib.

9. A medical device carrier according to any preceding claim, wherein the or each holding shoulder provides for pulling a medical device endoluminally through a patient during deployment.

10. An introducer assembly including:
a medical device carrier for holding an implantable medical device for deployment in a patient, the carrier including:
a carrier element on which a medical device can be held, the carrier element including a longitudinal axis and an outer perimeter;
at least one rib extending radially outwardly from the perimeter of the carrier element and including a distal end and a holding shoulder at the distal end extending at an angle substantially perpendicular to the longitudinal axis of the carrier catheter; and
at least one trigger wire, wherein the trigger wire or wires pass through channels, bores or grooves in the rib or ribs.

11. An introducer assembly according to claim 10, including an enlarged diameter head facing the rib or ribs, the enlarged diameter head including one or more bores or recesses providing a tight fit of a trigger wire.

12. An introducer assembly according to claim 10 or 11, wherein the trigger wire or wires are substantially straight through an associated bore, channel or groove in the rib or ribs.

13. An introducer assembly according to any of claims 10 to 12, including a plurality of said ribs angularly spaced around the perimeter of the carrier element, said spacing providing recesses for receiving at least one portion of a medical device to be held on the carrier.

14. An introducer assembly according to any of claims 10 to 13, including a sheath arranged to overlie the medical device carrier and be movable longitudinally relative thereto; wherein the rib or ribs are preferably a snug fit in the sheath.

15. An introducer assembly according to any of claims 10 to 13, wherein the or each holding shoulder provides for pulling a medical device during deployment, the introducer assembly being provided with no pusher element.
